(19) Europäisches Patentamt
European Patent Office
Office européen des brevets

(11) EP 2 948 523 B1

(12) **EUROPÄISCHE PATENTSCHRIFT**

(45) Veröffentlichungstag und Bekanntmachung des Hinweises auf die Patenterteilung:
**07.03.2018 Patentblatt 2018/10**

(21) Anmeldenummer: **14714923.1**

(22) Anmeldetag: **24.01.2014**

(51) Int Cl.:
***C07C 211/05*** (2006.01) ***C07C 211/07*** (2006.01)
***C07C 305/04*** (2006.01) ***C09K 8/584*** (2006.01)
***C08L 71/02*** (2006.01) ***C08G 65/334*** (2006.01)

(86) Internationale Anmeldenummer:
**PCT/DE2014/000026**

(87) Internationale Veröffentlichungsnummer:
**WO 2014/114287 (31.07.2014 Gazette 2014/31)**

(54) **HOCHKONZENTRIERTE, WASSERFREIE AMINSALZE VON KOHLENWASSERSTOFF-ALKOXYSULFATEN UND VERWENDUNG UND VERFAHREN UNTER VERWENDUNG VON WÄSSRIGEN VERDÜNNUNGEN DERSELBEN.**

HIGHLY CONCENTRATED, WATER-FREE AMINE SALTS OF HYDROCARBON ALKOXYSULFATES AND USE AND METHOD USING AQUEOUS DILUTIONS OF THE SAME

SELS D'AMINES ANHYDRES, TRÈS CONCENTRÉS, D'ALKOXYSULFATES D'HYDROCARBURES ET UTILISATION ET PROCÉDÉ FAISANT INTERVENIR DES FORMES DILUÉES AQUEUSES DE CES SELS D'AMINES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **25.01.2013 DE 102013100789**

(43) Veröffentlichungstag der Anmeldung:
**02.12.2015 Patentblatt 2015/49**

(73) Patentinhaber: **SASOL Germany GmbH**
**20537 Hamburg (DE)**

(72) Erfinder:
- **JAKOBS-SAUTER, Britta**
  **40764 Langenfeld (DE)**
- **KALTWASSER, Uwe**
  **45770 Marl (DE)**
- **NAPIERALA, Heinz**
  **45699 Herten (DE)**
- **KOCH, Herbert**
  **46348 Raesfeld (DE)**
- **ENNEKING, Meinolf**
  **44627 Herne (DE)**

(74) Vertreter: **Müller Schupfner & Partner**
**Patent- und Rechtsanwaltspartnerschaft mbB**
**Schellerdamm 19**
**21079 Hamburg (DE)**

(56) Entgegenhaltungen:
**EP-A1- 0 656 416** **EP-A2- 0 167 337**
**US-A- 4 017 405** **US-A- 4 477 372**
**US-A- 4 703 797**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 948 523 B1

## Beschreibung

Die vorliegende Erfindung betrifft hochkonzentrierte, wasserfreie Aminsalze von Kohlenwasserstoffpolyalkoxysulfaten, wobei die Salze aus der Gruppe der substituierten Amine, vorzugsweise der Alkanolamine, gewählt sind. Die erhaltenen Produkte sind bei Raumtemperatur niedrigviskos und pumpfähig. Die Salze sind sehr hydrolysestabil, auch bei hohen Temperaturen.

Des Weiteren betrifft die Erfindung die Verwendung der Kohlenwasserstoffpolyalkoxysulfate in wässriger Verdünnung, insbesondere Lösungen zur Anwendung in Erdöllagerstätten, mit dem Ziel eine verbesserte Ölförderung zu erreichen, wie z.B. zum Tensidfluten unterirdischer Reservoirs oder zur sogenannten "wettability alteration" (Veränderung des Benetzungsverhaltens) bzw. zur Gewinnung von Kohlenwasserstoffen aus Teersänden oder anderen kohlenwasserstoffversehenen Oberflächen oder Materialien. Wobei im Verständnis dieser Anmeldung die Gewinnung der Kohlenwasserstoffen auch die Reinigung von diesen umfaßt, einzeln oder gemeinsam.

### *Stand der Technik*

Die Salze der Alkylpolyalkoxysulfate werden in den unterschiedlichsten Anwendungen wie zum Beispiel als Textil- und Lederhilfsmitteln, in der Metallbearbeitung, als Schmierstoffe oder Reiniger, in der Kosmetik, als Öl- und Gasfeldchemikalien, als Wasch- und Reinigungsmittel verwendet.

Die Verwendung von Alkylpolyalkoxysulfaten als Öl- und Gasfeldchemikalien ist beispielsweise aus der GB 2168095 A bekannt. Weitere Beispiele offenbarend Alkylpolyalkoxysulfate enthaltend Propoxygruppen sind die WO 2009/124922 und die WO 2011/110502 A1, wobei neben den Alkali- und Erdalkalisalzen auch die Ammoniumsalze ($NH_4^+$) von Alkylpolyalkoxysulfaten genannt werden.

Die Herstellung der Alkylpolyalkoxysulfate erfolgt nach dem Stand der Technik aus Anlagerungsprodukten des Ethylenoxids (EO) und/oder Propylenoxids (PO) und/oder eines höheren Alkylenoxids (AO) an natürliche und synthetische Alkohole durch Umsetzung mit zum Beispiel Chlorsulfonsäure oder mit gasförmigem Schwefeltrioxid oder anderen geeigneten Sulfierungsmitteln, in äquimolaren Mengen.

Dabei werden die Schwefelsäurehalbester der Polyalkoxylate erhalten, die dann mit Basen neutralisiert werden. Die Neutralisationsbasen werden in solchen Konzentration in Wasser zugesetzt, dass die Alkylpolyalkoxysulfate als wässrige Lösungen oder als Pasten anfallen. Üblicherweise liegen mit Alkalihydroxiden oder Ammoniak neutralisierten Alkylpolyalkoxysulfate in flüssiger Form als wässriger Verdünnung mit einer Konzentration von unter 30 Gew.% vor. Oberhalb von 30 Gew.% bilden die Produkte hochviskose Gelphasen aus. Neben dem hohen Wassergehalt dieser Lieferform unterliegen die Produkte der Gefahr der Verkeimung, der den Zusatz von Biozid bzw. Konservierungsmittel notwendig macht. Es gibt hochkonzentrierte Lieferformen der Alkalisalze mit einem Aktivgehalt zwischen 70 Gew.% und teilweise über 80 Gew. %, deren Viskosität in einem handhabbaren Bereich liegt, was die Pumpfähigkeit betrifft. Solche höher konzentrierten Produkte unterliegen verstärkt der Gefahr der Hydrolyse, die im sauren pH- Bereich autokatalytisch abläuft. Bei der Hydrolyse wird $SO_3$ vom Molekül abgespalten und bildet mit Wasser Schwefelsäure. Durch die gebildete Schwefelsäure erniedrigt sich der pH - Wert weiter und beschleunigt die Hydrolyse.

Nach dem Stand der Technik kann durch Zusatz von geeigneten Puffersubstanzen der pH - Wert über eine begrenzte Zeit in einem neutralen pH - Bereich gehalten werden, um so die Hydrolyse zu verzögern.

Erhöhte Temperaturen beschleunigen die Zersetzung (Hydrolyse) der Alkylpolyalkoxysulfate. So wird empfohlen, die Produkte bei Temperaturen möglichst unter 30°C zu transportieren oder auch zu lagern. Produkte, die tiefen Temperaturen ausgesetzt waren, wieder aufzuwärmen, gestaltet sich sehr zögerlich, weil punktuelle Überhitzung vermieden werden muss. Aufgrund der hohen Viskosität in Temperaturbereichen um 0°C können die Produkte nicht umgepumpt oder gerührt werden. Lokale Überhitzung, zum Beispiel durch Erwärmung mit Dampf oder elektrische Beheizung, muss vermieden werden, weil das zu sogenannten "sauren Nestern" führt. Lokale Überhitzung kann somit zur Zersetzung (Hydrolyse) des gesamten Lagerbehälters führen.

Die EP 0167337 A2 beschreibt Salze von $C_4$ bis $C_{10}$ basierten Alkylalkoxysulfaten, die auch hochkonzentriert als wässrige fließfähige Zubereitungen vorliegen können. Im Unterschied zu den vorgenannten Salzen werden solche mit höheren Alkyl-Kettenlängen als hochviskos offenbart.

Aus der EP 0656416 A1 sind Tensidkonzentrate als Basistenside für konzentrierte Flüssigformulierungen enthaltend Alkanoiaminsalze von Alkylpolyethoxysulfaten bekannt. Die Flüssigformulierungen finden Anwendung als Wasch- und Reinigungsmittel und sind bei 70°C fließfähig.

EP 0167337 A2 offenbart hoch konzentrierte fließfähige Fettalkohol-Polyalkoxysulfate, wobei die Fettalkohole verzweigte Fettalkohole mit 4 bis 11 Kohlenstoffatomen sind und das Gegenion auch ein Aminsalz sein kann, einschließlich Triethanolamin und Triisopropanolamin, allerdings nach Neutralisation mit 25-50 Gew.% eines Neutrallsationsmittels.

US 4477372 lehrt Alkylethersulfate, welche mittels Ammoniumsalzen neutralisiert sein können. Weder Propoxylate oder gemischte Ethoxylate / Propoxylate werden offenbart noch hochkonzentrierte Zusammensetzungen.

EP 0656416 A1 offenbart fließfähige Flüssigformulierungen von Tensiden mit Mono-, Di- bzw. Trialkanolammonium-Salzen von Alkylethersulfaten, wobei der Tensidgehalt über 70% liegt. Es handelt sich jeweils um Ethoxylate.

US 4703797 A sowie US 4017405 A offenbaren flüssige Tensidzusammensetzungen enthaltend Alkylethersulfate, alledings nicht mit Aminen neutralisiert. Auch werden weder hochkonzentrierte Tensidzusammensetzungen noch Propoxylate oder gemischte Ethoxylate / Propoxylate offenbart.

Es besteht daher Bedarf an hochkonzentrierten Tensidzusammensetzungen der vorgenannten Art, die über einen weiten Temperaturbereich fileßfähig sind und während Transport und Lagerung bei höheren Temperaturen nicht oder vermindert der Hydrolyse unterliegen. Gleichzeitig sollen Tensidzusammensetzungen zugänglich gemacht werden, die sich zur Verwendung in der Erdölförderung eignen.

***Zusammenfassung der Erfindung***

Gegenstand der Erfindung ist eine Zusammensetzung wie In den unabhängigen Ansprüchen beschrieben. Bevorzugte Ausführungsformen sind Gegenstand der Unteransprüche oder nachfolgend beschrieben.

Überraschenderweise wurden hochkonzentrierte, wasserfreie Tensidzusammensetzungen von Alkylpolyalkoxysulfaten gefunden, die bei 25°C ohne Zusatz von Lösemitteln fließfähig sind. Aufgrund der Abwesenheit von Wasser sind die Zusammensetzungen hoch hydrolysestabil. Die geringe Gefahr von Hydrolyse ermöglicht die Lagerung oder auch den Transport bei höheren Temperaturen und die Verwendung von Puffersystemen zur Stabilisierung des pH - Werten ist nicht mehr erforderlich.

Die erfindungsgemäßen hochkonzentrierten, wasserfreien Aminsalze der Alkylpolyalkoxysulfate lassen sich leicht mit Wasser verdünnen. Bei dem Verdünnungvorgang werden die aus dem Stand der Technik bekannten hochviskosen Gelphasen, wie sie bei mit wässrigen Lösungen von Alkalihydroxiden oder Ammoniak neutralisierten Alkylpolyalkoxysulfaten auftreten, nicht durchlaufen.

Überraschenderweise wurde gefunden, dass auch die wässrigen Zusammensetzungen obiger Salze, wie sie z.B. durch Verdünnen obiger hochkonzentrierter, wasserfreier Zusammensetzungen oder auch durch wässrige Herstellung zugänglich sind, bessere Temperaturstabilität aufweisen als die entsprechenden mit Alkalihydroxiden oder Ammoniak neutralisierten Salze der Alkylpolyalkoxysulfate.

Diese erhöhte Temperaturstabilität in wässriger Lösung findet sich auch bei solchen Amin-neutralisierten Alkylpolyalkoxysulfaten, die nicht in wasserfreier Form bei Raumtemperatur fließ- und pumpfähig sind.

Mit Alkalihydroxiden oder Ammoniak neutralisierte Salze der Alkylpolyalkoxysulfate mit einer Konzentration von zum Beispiel 10 Gew.% Aktivgehalt in wässriger Lösung sind bei Temperaturen von über 30°C nur wenige Tage stabil und hydrolisieren vollständig. Mit Aminen neutralisierte Salze der Alkylpolyalkoxysulfate sind über mehrere Wochen, teilweise mehrere Monate, bei über 30°C oder noch höheren Temperaturen, wie z.B. bei 70°C, stabil und zeigen geringere Hydrolysegeschwindigkeiten. Überraschenderweise wurde festgestellt, dass Aminsalze von Alkylpolyalkoxysulfaten die gleiche "Optimale Temperatur" bzw. "Optimale Salinität" aufweisen wie die entsprechenden Natriumsalze. Die Temperatur / Salinität, bei der ein Wasser-Öl-Tensid-System, enthaltend ggf. weitere Zusätze, den "optimalen" Winsor III Zustand erreicht, nennt man Optimale Temperatur bzw. Optimale Salinität.

***Detaillierte Beschreibung der Erfindung.***

Die fließfähigen Aminsalze von Kohlenwasserstoffpolyalkoxysulfaten

$$R^4\text{-[EO, PO, AO]}_n\text{ -O-}SO_3^-\ HNR^1R^2R^{3+},$$

hiernach vereinfachend auch Alkylpolyalkoxysulfat - Salze genannt, enthalten eine oder mehrere primäre, sekundäre oder tertiäre Alkyl- und/oder Alkanol-Amin - Verbindungen.

Im Sinne der vorliegenden Erfindung stellen die angegebenen Zahlenwerte der Alkoxygruppen stets einen Mittelwert (Zahlenmittel) darstellen.

Geeignete, protonierte Alkyl-, Alkenyl- und/oder Alkanol-Amine sind:

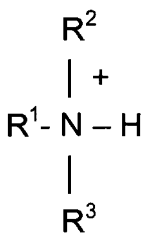

worin ein, zwei oder drei der Reste $R^1$, $R^2$ und $R^3$ sind unabhängig voneinander ausgewählt aus der Gruppe:

- Alkyl mit 1 bis 14, insbesondere 4 bis 8, Kohlenstoffatomen
- Alkenyl mit 3 bis 18, insbesondere 4 bis 8, Kohlenstoffatomen,
- Hydroxyalkyl mit 3 oder 4, insbesondere 3, Kohlenstoffatomen,
- für zwei oder drei der Reste $R^1$, $R^2$ und $R^3$ Hydroxyalkyl mit 2 Kohlenstoffatomen, wobei dann maximal einer der Reste $R^1$, $R^2$ und $R^3$ gleich H ist,
- und deren Gemischen,

wobei das Hydroxyalkyl jeweils ggf. alkoxyliert ist und wobei die verbleibenden Reste jeweils Wasserstoff sind. Auch umfasst sind Mischungen von Alkylpolyalkoxysulfat - Salzen mit verschiedenen Alkyl-, Akenyl- und Hydroxyalkyl-Resten.

Besonders geeignete Amin - Verbindungen sind zum Beispiel Mono- oder DiEthylamin, Mono- oder Di-Butylamin, Mono- oder Di-Oleylamin, Mono- oder Di-2-Ethylhexylamin oder sonstige Gemische. Beispiele für Alkanolamine sind Diethanolamin (DEA), Triethanolamin (TEA), Monoisopropanolamin (MIPA), Diisopropanolamin (DIPA) oder Triisopropanolamin (TIPA).

$R^4$ steht für ein oder mehrere, ggf. unterschiedliche C10 bis C36 Kohlenwasserstoff-reste, insbesondere C12 bis C24. Die zugrunde liegenden Alkohole können zum Beispiel Decanol, Undecanol, Dodecanol,Tridecanol, Tetradecanol, Pentadecanol, Oktadecanol, Behenylalkohol sowie die entsprechenden verzweigten ggf. auch ungesättigten Typen gleicher C - Kette wie zum Beispiel Oleylalkohol, 2-Hexyldecanol, 2-Hexyldodecanol, 2-Decyltetradodecanol oder Isotridecanol und deren Gemische sein. Die Alkohole können petrochemischen, oleochemischen oder synthetischen Ursprungs sein. Bespiele für synthetischen Ursprung sind Fischer-Tropsch-Alkohole, Guerbet-Alkohole oder Ziegler-Alkohole oder über die Alkene durch Hydroformylierung zugängliche Alkohole. Die umzusetzenden Alkene sind z.B. durch Metathese oder Oligomerisation verfügbar.

Alkypolyalkoxylate können aus Alkoholen hergestellt werden, indem diese mit Propylenoxid und ggf. Ethylenoxid und/oder einem höheren Alkylenoxid in beliebiger Reihenfolge umgesetzt werden. Die Umsetzung kann mit einem einzelnen Alkylenoxid oder zur Herstellung von Blöcken mit mehreren Alkylenoxiden nacheinander erfolgen. Ebenso ist es möglich, Gemische von Alkylenoxiden unterschiedlicher Zusammensetzung umzusetzen oder die Herstellung von Blöcken mit quasistatistisch verteilen Sequenzen, wie sie sich durch die Reaktionskinetik ergeben, zu kombinieren.

Die Anzahl der Alkoxygruppen EO, PO und AO beträgt 1 bis 16, insbesondere 2 bis 16 und besonders bevorzugt 3 bis 16 oder auch 4 bis 13, wobei die Nebenprodukte, welche keine Alkoxygruppen inkorporieren (n=0), nicht zur Mittelwertbildung beitragen. Die Alkoxygruppen sind aus:

- 1 bis 16, insbesondere 3 bis 16 oder auch 4 bis 13 oder 3 bis 10, Propoxygruppen (PO),

und ggf. zusätzlich aus einer oder mehreren der folgenden Gruppen

- 0 bis 15, insbesondere 1 bis 8 oder 1 bis 3, Ethoxygruppen (EO), und / oder
- 0 bis 10, insbesondere 0 bis 5 oder auch 1 bis 3, höhere (C4-C12) Alkoxygruppen (AO)

ausgewählt und sind statistisch verteilt, liegen in Blöcken vor oder beides. 0 bedeutet, dass auch keine der obigen Alkoxygruppen eingebaut sein können. Im Ergebnis bedeutet dies, dass bei Kohlenwasserstoffpolyalkoxysulfaten mit gemischten Alkoxylat-Gruppen stets zumindest eine Propoxygruppe eingebaut sein muss oder, daß das Kohlenwasserstoffpolyalkoxysulfat ausschließlich Propoxygruppen enthält.

Die Umsetzung des Alkohols mit Alkylenoxiden erfolgt katalytisch. Als Katalysatoren können klassische Basen, wie zum Beispiel NaOH, KOH, Natriummethylat, oder auch Doppelmetallcyanid (DMC) Katalysatoren verwendet werden. Durch gezielte Verwendung der Katalysatoren können Produkteigenschaften der Alkylpolyalkoxylate oder auch der Alkylpolyalkoxysulfate eingestellt werden, was in den unterschiedlichsten Anwendungen vorteilhaft genutzt werden kann.

Die Sulfatierung der Alkylpolyalkoxylate kann in für Fettalkoholethersulfate an sich bekannter Weise mit den üblichen Methoden erfolgen, wobei die Verwendung von Fallfilmreaktoren bevorzugt ist. Als Sulfiermittel kommen z.B. Oleum, Chlorsulfonsäure oder insbesondere Schwefeltrioxid in Frage, wobei letzeres insbesondere mit einem Inertgas verdünnt zum Einsatz kommt. Der entstehende Schwefelsäurehalbester ist nicht stabil und muss daher unverzüglich in einen Neutralisationskreislauf überführt werden, in dem er mit entsprechenden wasserfreien Aminen, insbesondere Alkanolaminen, unter hoher Scherung umgesetzt bzw. neutralisiert wird. In der bevorzugten Ausführungsform wird die Temperatur bei der Neutralisation bei 45 bis 65°C, insbesondere 50 bis 60°C, gehalten, bei einem pH-Wert (bezogen auf 1 Gew.% des Produktes in Wasser) von pH 7,5 bis 10 (nach DIN-EN 1262:2004).

Durch Neutralisation mit geeigneten Alkyl-/Alkanol-Aminverbindungen wird eine wasserfreie und ggf. lösemittelfreie, bei 25°C (Raumtemperatur) fließfähige Zusammensetzung erhalten. Die Alkylpolyalkoxysulfonsäure ist vorzugsweise äquimolar oder mit einem geringen Überschuss von Amin-Verbindungen abzumischen, wobei der pH - Wert auf

einen neutralen bis schwach alkalischen Wert eingestellt wird, was mit einem 0,1 bis 5 molprozentigen Überschuss, vorzugsweise 0,1 bis 2 molprozentigen Überschuss, erreicht wird.

Das so erhaltene hochkonzentrierte Aminsalz der Alkylpolyalkoxysulfate enthält zu kleineren Anteilen Alkohole, Alkylenglykole(auch sulfatiert), Alkylpolyalkoxylate oder auch andere Nebenprodukte. Der Anteil an unsulfatierten Material (nichtionische Substanzen) im Endprodukt beträgt typischerweise 0,1 bis 10 Gew.%, vorzugsweise 0,5 bis kleiner 5 Gew.% (bestimmt nach DIN EN 13273).

Der Gehalt an erfindungsgemäßen Salzen von Alkylpolyalkoxysulfaten in der Zusammensetzungen beträgt größer 85 Gew.%, insbesondere größer 90 Gew.%, vorzugsweise größer 95 Gew.%.

Fließfähig bei 25°C im Sinne der Erfindung bedeutet, dass die erhaltenen Zubereitungen eine Viskosität von kleiner 20 000 mPas, vorzugsweise kleiner 10 000 mPas, bei einer Temperatur von 25°C und einer Scherrate von D= $10s^{-1}$ aufweisen. Die Viskosität wird mit einem Rheometer mit Kegel/Platte - Meßgeometrie nach DIN 53019 bestimmt. Fließfähig bei einer anderen Temperatur, z.B.15°C, bedeutet, im Sinne der Erfindung, dass die gleichen Werte für die Viskosität bei der entsprechenden anderen Temperatur, z.B.15°C, eingehalten werden.

Ist gewünscht, die Viskosität der Alkyl-/Alkanol-Aminsalze der Alkylpolyalkoxysulfate weiter herabzusetzen, kann dies durch Zugabe geeigneter Lösemittel (abgesehen von Wasser) wie zum Beispiel Glykolen, z.B. Ethandiol, 1,2-Propandiol, anderen Polyolen oder deren Gemischen erfolgen.

Zur Bestimmung der Lagerstabilität (Hydrolysestabilität) werden die Produkte in Glasbehälter gefüllt, der überstehende Gasraum wird mit Stickstoff gespült und luftdicht verschlossen. Mehrere verschlossene Behältnisse, gefüllt mit einem Produkt, werden bei den entsprechenden Temperaturen in einem handelsüblichen Heizschrank gelagert. Nach einer bestimmten Zeit werden die Behältnisse entnommen und die pH-Wert-Änderung und die Säurezahl, mittels Titration in mg KOH/g, bestimmt.

Die erfindungsgemäßen wasserfreien Aminsalze der Alkylpolyalkoxysulfate zeigen bei Lagerung über 50°C oder sogar 70°C nach 3 Monaten Lagerzeit, oder sogar 6 Monaten, kein Absinken des pH-Werts unter 6, folglich eine besonders gute Hydrolysestabilität. Mit Alkalilaugen und Ammoniak neutralisierte, wässrige Salze der Alkylpolyalkoxysulfate unterliegen im Unterschied bei Lagerung von 30°C bereits nach 7 bis 14 Tagen der Hydrolyse, der pH-Wert sinkt innerhalb dieser Zeit auf Werte unter pH 3, teilweise unter pH 2, ab.

Die erfindungsgemäßen hochkonzentrierten, wasserfreien und ggf. lösemittelfreien Aminsalze der Alkylpolyalkoxysulfate lassen sich leicht mit Wasser verdünnen. Bei dem Verdünnungsvorgang treten überraschender Weise keine hochviskosen Gelphasen auf, wie es bei der Verdünnung von 70%igen wässrigen Zubereitungen von mit Alkalihydroxiden oder Ammoniak neutralisierten Alkylpolyalkoxysulfaten der Fall ist. Die Verdünnung der erfindungsgemäßen hochkonzentrierten, wasserfreien Aminsalze der Alkylpolyalkoxysulfate mit Wasser ist bei 35 bis 45 °C, insbesondere etwa 40°C, besonders schnell und mit geringen Energieeintrag verbunden, d.h. einfach durchführbar unter Rühren mit geringer Schergeschwindigkeit. Dies stellt einen erheblichen Vorteil dar, der die Verwendung von aufwendigen Verdünnungsanlagen oder speziellen Mischern überflüssig macht.

Zur Bestimmung der Verdünnbarkeit wird das Produkt bei 25°C in solchen Verhältnissen mit Wasser gemischt, dass sich entsprechende Lösungen mit definierten anionischen Aktivgehalten ergeben. Dies erfolgt durch Zugabe von Tensid in destilliertes Wasser bei 25°C unter Rühren von Hand mit einem Spatel oder Glasstab. Geschieht dies, ohne dass hochviskose Gelphasen durchlaufen werden, die von Hand nicht mehr rührbar oder mischbar sind, ist das Produkt definitionsgemäß leicht verdünnbar.

Der niedrige Pourpoint von unter 10°C, insbesondere unter 0°C, erlaubt die Lagerung oder auch den Transport bei niedrigen Temperaturen unter Erhalt des fließfähigen Zustands. Der Pourpoint der erfindungsgemäßen Alkyl-/Alkanol-Aminsalze der Alkylpolyalkoxysulfate wird nach ASTM D97-09 ermittelt, indem das Produkt in 3°C - Schritten abgekühlt wird. Wenn nach 10 Minuten bei einer bestimmten Temperatur das Produkt nach Kippen des Behältnisses in die Waagerechte innerhalb von 5 Sekunden nicht fließt, gilt der 3°C höhere Wert als der Pourpoint.

Die erfindungsgemäßen hochkonzentrierten, wasserfreien und ggf. lösemittelfreien Aminsalze der Alkylpolyalkoxysulfate sind nach Verdünnung mit Wasser aufgrund ihrer im Vergleich mit den Alkali- oder Ammoniumsalzen höheren Stabilität, insbesondere Temperaturstabilität, vorteilhaft zur Anwendung in Erdöllagerstätten, mit dem Ziel einer verbesserten Ölförderung, einsetzbar, wie z.B. zum Tensidfluten unterirdischer Reservoirs oder zur sogenannten "wettability alteration" (Veränderung des Benetzungsverhaltens) bzw. zur Gewinnung von Kohlenwasserstoffen aus Teersänden oder anderen kohlenwasserstoffversehenen Oberflächen oder Materialien.

Die erhöhte Temperaturstabilität in wässriger Lösung findet sich auch bei solchen Amin-neutralisierten Alkylpolyalkoxysulfaten, die nicht in wasserfreier Form bei Raumtemperatur fließ- und pumpfähig sind. Diese sind aber ebenso geeignet zur zur Anwendung in Erdöllagerstätten, mit dem Ziel einer verbesserten Ölförderung bzw. zur Gewinnung von Kohlenwasserstoffen aus Teersänden oder anderen kohlenwasserstoffversehenen Oberflächen oder Materialien.

Unter primärer Erdölförderung versteht man die Förderung des Erdöls durch den Eigendruck im Reservoir. Mittels der primären Förderung lassen sich je nach Lagerstätte oft nur ca. 5 bis 10% der in der Lagerstätte vorhandenen Erdölmenge fördern bis der Eigendruck nicht mehr zur Förderung ausreicht.

Bei der sekundären Förderung wird Flüssigkeit in die Lagerstätte eingepresst, um den Druck aufrechtzuerhalten

oder wieder zu erhöhen. Durch Einpressen des Wassers durch sogenannte Injektionsbohrlöcher wird das Erdöl durch die Hohlräume in der Formation langsam in Richtung der Produktionsbohrung gedrückt. So lange wie die Hohlräume vollständig mit Öl gefüllt sind, wird das viskosere Öl durch das Wasser vor sich hergeschoben. Sobald das dünnflüssige Wasser durch Hohlräume durchbricht, strömt es ab diesem Zeitpunkt auf dem Weg des geringsten Widerstandes, also durch den gebildeten Kanal, und schiebt nicht mehr das Öl vor sich her. Die unterschiedliche Polarität von Öl und Wasser sorgt für eine hohe Grenzflächenenergie bzw. Grenzflächenspannung. Daher nehmen beide zueinander die kleinste Kontaktfläche ein, was in einem kugelförmigen Öltropfen resultiert, welcher nicht mehr durch die feinen Kapillaren in der Lagerstätte passt. Das Öl ist in diskontinuierlicher Form (vereinzelte Kugeltropfen) in den Kapillaren gefangen. Primäre und sekundäre Förderung können im Regelfall nur ca. 20 bis 40 % der in der Lagerstätte vorhandenen Erdölmenge fördern.

Enhanced Oil Recovery (abgekürzt EOR) bzw. Improved Oil Recovery (abgekürzt IOR) bzw. im Deutschen tertiäre Erdölförderung, nachfolgend kurz (gemeinsam) EOR genannt, bezieht sich auf Techniken zur Erhöhung der Menge an rohem Erdöl, die aus einem Reservoir, z.B. ein Ölfeld, extrahiert werden können. EOR kann auch als verbesserte Ölgewinnung im Vergleich zu einer lediglich primären oder einer primären und sekundäre Ölgewinnung bezeichnet werden. Mit EOR können typischerweise ca. 40% - 60% des nach der primären Förderung verbliebenen Rohöls aus dem Reservoir extrahiert werden.

EOR kann durch eine Vielfalt von Verfahren, wie zum Beispiel mischbaren Gasinjektion (einschließlich Kohlendioxidinjektion), Chemikalieninjektion (einschließlich Polymerfluten und/oder alkalisches Fluten und/oder Tensidfluten oder Kombinationen daraus, einschließlich "wettability alteration" (Änderung der Benetzbarkeit von Gesteinsoberflächen), und Kohlendioxid-Schaum, mikrobielle Injektion oder thermisches "Recovery" (welche cyclische Dampfinjektion umfasst), Dampffluten und Feuerfluten, erreicht werden. Weiterhin können z.B. Öl- oder Teersände oder andere ölbenetzte Oberflächen durch Behandlung mit wässrigen Lösungen der Amin-neutralisierten Alkylpolyalkoxysulfate entölt werden.

Die Injektion von alkalischen wässrigen Lösungen in Reservoirs, dessen Rohöl natürlich vorkommende organische Säuren enthält, hat die Bildung von Seifen zur Folge. Diese Seifen senken die Grenzflächenspannung und können somit die Produktion erhöhen. Einige Rohöle enthalten Carbonsäuren mit beispielsweise C11 bis C20-Alkylketten, naphthenische Säuren und andere. Eine Verbesserung der Produktion solcher "reaktiver" Öle kann unter Verwendung von Lauge (zum Beispiel NaOH oder $Na_2CO_3$) in einer Tensidzusammensetzung erzielt werden. Die Injektion einer verdünnten Lösung eines wasserlöslichen Polymers, das die Viskosität des injizierten Wassers erhöht und an die Viskosität des Rohöls in der Formation angleicht, kann die Produktion von Öl aus geologischen Formationen ausreichender Permeabilität erhöhen.

Für Reservoirs niedriger Permeabilität ("tight formations") bietet sich hingegen beispielsweise die Methode der sogenannten "wettability alteration" zur erweiterten Erdölförderung an. Dabei wird mit Hilfe von Tensiden, die in verdünnter wässiger Lösung injiziert werden, die Benetzbarkeit des Gesteins von ölbenetzt zu wasserbenetzt geändert, womit weiteres Öl mobilisiert wird.

Auf das Erdöl wirken viskose und kapillare Kräfte, wobei das Verhältnis dieser beiden Kräfte zueinander die mikroskopische Ölentfernung bestimmt. Mittels eines dimensionslosen Parameters, der sogenannten Kapillarzahl N, wird das Wirken dieser Kräfte beschrieben. Sie ist das Verhältnis der Viskositätskräfte (Geschwindigkeit x Viskosität der drückenden Phase) zu den Kapillarkräften (Grenzflächenspannung zwischen Öl und Wasser x Benetzung des Gesteins):

$$N = \mu V / \sigma \cos\theta$$

$\mu$ ist die Viskosität des Erdöl mobilisierenden Fluids, V die Darcy-Geschwindigkeit (Durchfluss pro Flächeneinheit), $\sigma$ die Grenzflächenspannung zwischen Erdöl mobilisierender Flüssigkeit und Erdöl und $\theta$ der Kontaktwinkel zwischen Erdöl und dem Gestein (C. Melrose, CF. Brandner, J. Canadian Petr. Techn. 58, Oct. Dez., 1974). Je höher die Kapillarzahl, desto größer die Mobilisierung des Öls und somit auch der Entölungsgrad.

Es ist bekannt, dass die Kapillaritätszahl gegen Ende der sekundären Erdölförderung im Bereich von etwa $10^{-6}$ liegt und dass es notwendig ist, die Kapillaritätszahl auf etwa $10^{-3}$ bis $10^{-2}$ zu erhöhen, um zusätzliches Erdöl mobilisieren zu können.

Man kann zum Beispiel die Grenzflächenspannung $\sigma$ zwischen dem Erdöl und der wässrigen Phase durch den Zusatz von geeigneten Tensiden absenken, auch als "Tensidfluten" bekannt. Hierzu eignen sich insbesondere Tenside, welche $\sigma$ auf Werte von höchstens 0,01 mN/m (ultralow interfacial tension) herabsetzen können.

Spezielle Formulierungen von Tensiden mit Wasser und Öl bilden eine Mikroemulsion (Winsor Typ III). Das Auftreten bestimmter Phasenzustände wird durch interne (Zusammensetzung) und externe Parameter (wie Temperatur und Salinität) bestimmt, wobei letztere in der Regel durch die geologischen Bedingungen in einem Ölreservoir vorgegeben

sind. Der Winsor III Phasenzustand, auch dreiphasige Mikroemulsion genannt (wobei die eigentliche Mikroemulsion die Mittelphase ist, begleitet von einer Wasser- und einer Öl-Überschussphase), zeichnet sich durch extrem niedrige Grenzflächenspannungen (IFT) aus. Daher wird dieser Zustand auch als "optimal" bezeichnet und die zugehörigen Parameter als "Optimale Salinität" bzw. "Optimale Temperatur".

Überraschenderweise zeigen die Aminsalze der Alkylpolyalkoxysulfate in Bezug auf die EOR-Anwendung die gleichen OS*/OT* Paare (OT* = Optimale Temperatur, OS* = Optimale Salinität) wie die korrespondierenden Natriumsalze, d.h. sie erreichen unter den gleichen Reservoirbedingungen den optimalen Zustand mit ultraniedriger Grenzflächenspannung. Die Mittelphase ist in der Regel niedrig viskos. Eine niedrige Viskosität ist wünschenswert zum Transport der Emulsion in der Erdölformation.

Üblich sind Lagerstättentemperaturen von ca. 30°C bis ca. 130°C in Gegenwart von stark salzhaltigem Wasser. Wenn das zur Verfügung stehende Wasser reich an Calcium- und Magnesiumionen ist, kann das zugesetzte Alkali eine Ausfällung von Kationen verursachen, wie $Ca^{+2}$ oder $Mg^{+2}$. Um eine solche Ausfällung zu verhindern, wird der Zusatz von Chelatbildern, wie zum Beispiel EDTA zu der Tensidzusammensetzung, erforderlich. Alternativ können auch Wasserenthärtungsprozesse verwendet werden, um das Injektionswasser aufzuarbeiten. Alternativ können auch Tenside verwendet werden, die in stark salzhaltigem Lagerstättenwasser (Injektionswasser) löslich sind.

Für die Anwendung in der tertiären Erdölförderung ist eine hohe Langzeitstabilität der Tenside unter Lagerstättenbedingungen notwendig, da die Wanderungsgeschwindigkeit in der Formation oft weniger als 1 m/Tag beträgt. Je nach dem Abstand zwischen Injektions- und Förderbohrung können daher die Verweilzeiten des Tensids in der Erdöllagerstätte mehrere Monate betragen.

***Versuchsbeispiele***

Beispiel a

Ein verzweigter primärer C12/C13 Alkohol (ISALCHEM® 123) wird mit KOH als Katalysator und 8 Mol Propylenoxid bei Temperaturen von 130 - 165°C und einem Druckbereich von 2 bis 3 bar in einem Rührautoklaven umgesetzt. Das erhaltene Alkoxyd (ISALCHEM® 123 + 8 PO (OH Zahl: 83.2 mg KOH/g, Wasser: 0.03 %, Molekulargewicht: 674.3 g/mol) wurde in einer kontinuierlichen Sulfierungsapparatur sulfiert (Fallfilmreaktor der Firma BALLESTRA). An einem $V_2O_5$ Katalysator wurde bei hoher Temperatur gasförmiges $SO_2$ zu $SO_3$ umgesetzt. Das Gas wurde abgekühlt und mit Luft (Taupunkt -60°C) verdünnt. Der Anteil von $SO_3$ in der Luft betrug 7 Vol.%.

In einem Fallfilmreaktor mit Verteiler wurde der propoxylierte Alkohol mit dem $SO_3$/Luftgemisch zur Reaktion gebracht. Das Reaktionsgas durchströmt den Fallfilmreaktor mit hoher Geschwindigkeit und erzeugt beim Kontakt mit dem propoxylierten Alkohol hohe Turbulenzen. Dadurch wurde ein intensiver Stoffaustausch generiert. Intensive Kühlung des Fallfilmreaktors sorgt für die Abfuhr der Reaktionswärme. Am Ausgang des Fallfilmreaktors wurde die Gas- / Flüssigkeitstrennung durchgeführt. Die flüssige Phase gelangt in die Neutralisation, die Gasphase in die Abgasaufarbeitung.

MIPA als Neutralisationsmittel wurde kontinuierlich in stöchiometrischen Mengen zugeführt. Gleichzeitig wird das Produkt im Kreislauf durch ein hoch scherendes Mischwerkzeug homogenisiert. Aus dem Neutralisationskreislauf wurde das fertige Produkt kontinuierlich entnommen. Beispiele b-k wurden gemäß obiger Versuchsbeschreibung hergestellt zu den entsprechenden Alkoholpropoxysulfatsalzen umgesetzt:

b) ein Sulfat eines verzweigten primären C12/C13 Alkohols (ISALCHEM® 123) mit durchschnittlich 8 Propoxyl-Gruppen mit TIPA

c) ein Sulfat eines verzweigten primären C12/C13 Alkohols (ISALCHEM® 123) mit durchschnittlich 8 Propoxyl-Gruppen mit mono-Octylamin,

d) ein Sulfat eines verzweigten primären C14/C15- Alkohols (ISALCHEM® 145) mit durchschnittlich 4 PO Gruppen mit MIPA,

e) ein Sulfat eines verzweigten primären C14/C15- Alkohols (ISALCHEM® 145) mit durchschnittlich 4 PO Gruppen mit TIPA,

f) ein Sulfat eines verzweigten primären C14/C15- Alkohols (ISALCHEM® 145) mit durchschnittlich 4 PO Gruppen mit DEA,

g) ein Sulfat eines teilweise verzweigten primären C16/C17- Alkohols (LIAL® 167) mit durchschnittlich 4 PO Gruppen mit MIPA,

h) ein Sulfat eines linearen C12/C14- Alkohols mit durchschnittlich 9 PO Gruppen mit MIPA und

i) ein Sulfat eines linearen C12/C14- Alkohols mit durchschnittlich 7 PO Gruppen mit TIPA.

Als Vergleichsbeispiele wurden gemäß obiger Versuchsbeschreibung hergestellt:

j) ein verzweigter C24 Alkohol (ISOFOL® 24) (ohne PO Gruppen) mit MIPA und

k) ein verzweigter C24 Alkohol (ISOFOL®24) (ohne PO Gruppen) mit TIPA.

Tabelle 1

| Beispiel | anionische Aktivsubstanz (Gew. %) | unsulfierte Materie (Gew. %) | Amin - Sulfat (Gew. %) | Freies Amin (Gew. %) | pH1 %-ig in Wasser | Viskosität (mPas, 25°C und 10 $s^{-1}$) |
|---|---|---|---|---|---|---|
| a) | 96,5 | 1,3 | 1,2 | 0,9 | 9 | 2450 |
| b) | 93,7 | 3,4 | 0,9 | 1,9 | 7,2 | 15000 |
| c) | 96,2 | 1,4 | 0,8 | 1,6 | 8,8 | 850 |
| d) | 96 | 3,5 | 0,3 | 0,2 | 7,9 | 3400 |
| e) | 92,7 | 2,5 | 0,9 | 3,8 | 7,5 | 19000 |
| f) | 93,9 | 4,5 | 1 | 0,6 | 7,5 | 7400 |
| g) | 96,6 | 2,5 | 0,5 | 0,4 | 8,5 | 3150 |
| h) | 94,8 | 2,9 | 1,4 | 0,9 | 8,6 | 1700 |
| i) | 89,3 | 3,5 | 0,9 | 6,2 | 7,7 | 9000 |
| j) | 95 | 3,1 | 1,7 | 0,2 | 8,3 | fest |
| k) | 90,8 | 5,1 | 3,8 | 0,3 | 7,8 | 75000 |

Analoge Verbindungen der Beispiele a-k, die mit NaOH anstelle der erfindungsgemäßen Amine neutralisiert und anschließend entwässert wurden, sind bei 25°C Feststoffe. Die erhaltenen Zusammensetzungen und Eigenschaften finden sich in der folgenden Tabelle 1.

In Tabelle 2 wurden die pH-Werte von 10%igen wässrigen Lösungen der aufgeführten Verbindungen vor und nach Lagerung bei 70°C bestimmt. Das Ausmaß der pH-Werterniedrigung ist ein Indiz für die Hydrolyse.

Tabelle 2

| **Zusammensetzung** | **Start** | **70°C** | |
|---|---|---|---|
| | **pH-Wert** | **Lagerzeit** | **pH-Wert** |
| gemäß Beispiel a) | 8,5 | 6 Monate | 6,5 |
| gemäß Beispiel b) | 6,9 | 6 Monate | 6,1 |
| Natriumsalz eines verzweigter primärer $C_{12}/C_{13}$ Alkohol (ISALCHEM® 123) mit durchschnittlich 8 Propoxyl-Gruppen | 8,1 | 2 Wochen | 1,4 |
| Natriumsalz eines linearen $C_{12}/C_{14}$ Alkohols mit durchschnittlich 7 PO Gruppen | 10,1 | 2 Wochen | 1,3 |
| Natriumsalz eines linearen $C_{10}$ Alkohols mit durchschnittlich 4 PO und 1 EO Gruppe | 10,2 | 7 Wochen | 1,6 |

Um Aussagen über die Langzeitstabilität unter simulierten Anwendungsbedingungen zu machen, wurden Formulierungen wie folgt hergestellt:

- jeweils 1 % Tensid bezogen auf aktive Substanz in voll entsalztem Wasser
- + 0,1 % Polymer "Flopaam® 3330 S" (SNF SAS - Frankreich)
- + 2% NaCl und
- Einstellen auf einen pH-Wert von pH 10,0 mit NaOH oder mit $Na_2CO_3$
- Halten bei einer definierten Temperatur über das unten angegebene Zeitintervall und
- Messen des pH Werts als Indiz für die Zersetzung des Alkylpolyalkoxysulfates / Alkoholethersulfates.

Die Ergebnisse sind in den Tabellen 3 und 4 zusammengestellt.

Tabelle 3

| | Salz | 70 °C Na2CO3 (pH Wert bei Start: 10.0) |
|---|---|---|
| Sulfat eines verzweigten primären C16/C17 Alkohols (Lial ® 167) mit durchschnittlich 4 Propoxyl-Gruppen | Na | after 2 Wochen pH 4.8 |
| | MIPA | after 6 Monaten pH 8.9 |
| | TIPA | nach 6 Monaten pH 9.8 |
| Sulfat eines verzweigten primären C12/C13 Alkohols (ISALCHEM® 123) mit durchschnittlich 8 Propoxyl-Gruppen | Na | nach 3 Wochen pH 5.8 |
| | MIPA | nach 6 Monaten pH 9.4 |
| | TIPA | nach 6 Monaten pH 9.7 |
| Sulfat eines verzweigten primären C14/C15 Alkohols (ISALCHEM® 145) mit durchschnittlich 4 PO Gruppen | Na | nach 2 Wochen pH 5.0 |
| | MIPA | nach 6 Monaten pH 9.8 |
| | TIPA | nach 6 Monaten pH 9.8 |
| Sulfat eines lineares C12 Ziegler Alkohols mit durchschnittlich 7 PO Gruppen | Na | nach 3 Wochen pH 5.6 |
| | MIPA | nach 6 Monaten pH 8.7 |
| | TIPA | nach 6 Monaten pH 9.8 |

Tabelle 4

| | Salz | NaOH (pH Wert bei Start: 10,0) | $Na_2CO_3$ (pH Wert bei Start: 10,0) |
|---|---|---|---|
| Sulfat eines verzweigten primären C16/C17 Alkohols (Lial ® 167) mit durchschnittlich 4 Propoxyl-Gruppen | Na | nach 5 Wochen bei 70°C pH 5,8 | nach 8 Wochen bei 70°C pH 5,3 (+ Antioxidanz) |
| | MIPA | nach 19 Wochen bei 80°C pH 8,8 | nach 19 Wochen bei 80°C pH 9,7 |

## Patentansprüche

**1.** Hochkonzentrierte und bei 25°C fließfähige Zusammensetzung von Aminsalzen von Kohlenwasserstoffpolyalkoxysulfaten aufweisend:

(A) größer 80 Gew.%, insbesondere größer 90 Gew.%, insbesondere größer 95 Gew.%, Aminsalze von Kohlenwasserstoffpolyalkoxysulfaten der Formel

$$R^4\text{-O-[EO, PO, AO]}_n\text{-SO}_3^-\text{HNR}^1\text{R}^2\text{R}^{3+}$$

wobei

- ein, zwei oder drei der Reste $R^1$, $R^2$ und $R^3$ sind unabhängig voneinander ausgewählt aus der Gruppe:

  - Alkyl mit 1 bis 14 Kohlenstoffatomen,,
  - Alkenyl mit 3 bis 18 Kohlenstoffatomen,
  - Hydroxyalkyl mit 3 oder 4 Kohlenstoffatomen,
  - zwei oder drei Reste sind Hydroxyalkyl mit 2 Kohlenstoffatomen,
  - und deren Gemischen,

  wobei das Hydroxyalkyl ggf. alkoxyliert ist und
  wobei die verbleibenden Reste jeweils Wasserstoff sind; und
- $R^4$ ein oder mehrere unterschiedliche C10 bis C36 Kohlenwasserstoff(e) ist/sind;

ggf. in Mischung mit weniger als 20 Gew.%, insbesondere weniger 10 Gew.%, von obigen Aminsalzen von Kohlenwasserstoffpolyalkoxysulfaten, in denen die Alkoxygruppen ausschließlich EO sind;
(B) zumindest 0,1 bis zu kleiner 5 Gew.%, insbesondere 0,2 bis 3 Gew.%, des nicht sulfatierten polyalkoxylierten Kohlenwasserstoffs $R^4$-O-[EO,PO,AO]$_n$-H oder Gemisches;
(C) bis zu kleiner 5 Gew.% des nicht-sulfatierten Hydroxy-Kohlenwasserstoffs $R^4$-OH oder Gemisches;
wobei (B) und (C) zusammen 0,1 bis 10 Gew.%, insbesondere 0,5 bis 5 Gew.%, der Zusammensetzung ausmachen;

wobei die Zusammensetzung
kleiner 2 Gew.% Wasser aufweist und
die Anzahl n der Alkoxygruppen EO, PO, AO zusammen 1 bis 16, beträgt
und die Alkoxygruppen ausgewählt sind aus:

- 1 bis 16, Propoxygruppen (PO)
und ggf. einer oder mehrerer der Gruppen
- 0 bis 15 Ethoxygruppen (EO), und
- 0 bis 10 C4 bis C12 Alkylenoxygruppen (AO),

und die Alkoxygruppen statistisch verteilt sind oder in Blöcken vorliegen oder beides.;

**2.** Zusammensetzung gemäß Anspruch 1, worin $R^4$ für ein oder mehrere unterschiedliche C12 bis C36 Kohlenwasserstoffe, insbesondere C12 bis C24 Kohlenwasserstoffe steht.

**3.** Zusammensetzung gemäß zumindest einem der Ansprüche 1 oder 2, wobei zwei oder drei der Reste $R^1$, $R^2$ und $R^3$ Hydroxyalkyl mit jeweils 2 und/oder 3 Kohlenstoffatomen sind und die verbleibenden Reste jeweils Wasserstoff, wobei das C2 oder C3 Hydroxyalkyl ggf. alkoxyliert ist.

**4.** Zusammensetzung gemäß zumindest einem der Ansprüche 1 bis 3, wobei ein, zwei oder drei der Reste $R^1$, $R^2$ und $R^3$ jeweils Isopropanol sind, vorzugsweise ein oder drei der Reste $R^1$, $R^2$ und $R^3$, und die verbleibenden Reste jeweils Wasserstoff sind, wobei das Isopropanol ggf. alkoxyliert ist.

**5.** Zusammensetzung gemäß zumindest einem der Ansprüche 1 bis 4, wobei die Anzahl n der Alkoxygruppen EO, PO, AO zusammen 3 bis 16 vorzugsweise 4 bis 13 oder 3 bis 10 beträgt.

**6.** Zusammensetzung gemäß zumindest einem der Ansprüche 1 bis 5, wobei die EO, PO, AO Alkoxygruppen unabhängig voneinander ausgewählt sind aus:

- 4 bis 13 Propoxygruppen (PO),
- 1 bis 8 oder 1 bis 3, Ethoxygruppen (EO), und
- 0 bis 5 oder 1 bis 3, C4 bis C12 Alkylenoxygruppen (AO), insbesondere Butoxygruppen, und

die Alkoxygruppen statistisch verteilt sind oder in Blöcken vorliegen oder beides.

**7.** Zusammensetzung gemäß zumindest einem der Ansprüche 1 bis 6, wobei die Zusammensetzung aus den Komponenten (A) bis (C) und ggf. Wasser besteht.

**8.** Zusammensetzung gemäß zumindest einem der Ansprüche 1 bis 7, wobei die Zusammensetzung kleiner 0,5 Gew.% Wasser, besonders bevorzugt kleiner 0,05 Gew.% Wasser aufweist.

**9.** Zusammensetzung gemäß zumindest einem der Ansprüche 1 bis 8, enthaltend weiterhin

(D) bis zu 5 Gew.%, insbesondere 0,1 bis 5 Gew.%, des nicht-sulfatierten Amins bzw. Amingemisches $NR^1R^2R^3$.

**10.** Zusammensetzung gemäß zumindest einem der Ansprüche 1 bis 9, enthaltend weiterhin

(E) 0,1 bis 5 Gew.%, insbesondere 0,1 bis 2 Gew.%, das Sulfat des Amins bzw. Amingemisches $(HNR^1R^2R^3)_2SO_4$.

**11.** Zusammensetzung gemäß zumindest einem der Ansprüche 1 bis 10, enthaltend weiterhin

(F) 0,1 bis 5 Gew.%, insbesondere 0,5 bis 2 Gew.%, $HNR^1R^2R^{3+}$ $-O^3SO$-[EO, PO, AO]-$SO_3^-$ $HNR^1R^2R^{3+}$.

**12.** Zusammensetzung gemäß zumindest einem der Ansprüche 1 bis 11, enthaltend weiterhin 0,05 bis 10 Gew.%, insbesondere 0,1 bis 5 Gew.%, des sulfatierten nicht alkoxylierten Hydroxykohlenwasserstoffs $R^4$-O-$SO_3^-$ $HNR^1R^2R^{3+}$ oder Gemische.

**13.** Zusammensetzung gemäß zumindest einem der Ansprüche 1 bis 12, wobei die Zusammensetzung bei 15°C und höher fließfähig ist.

**14.** Zusammensetzung gemäß zumindest einem der Ansprüche 1 bis 13, wobei die Zusammensetzung einen Pourpoint von kleiner + 5°C, vorzugsweise kleiner - 5 °C, aufweist.

**15.** Zusammensetzung gemäß zumindest einem der Ansprüche 1 bis 14 enthaltend in der Summe weniger als 10 Gew.%, vorzugsweise weniger als 3 Gew.%, andere nicht-ionische Tenside, abgesehen von $R^4$-O-[EO,PO,AO]$_n$-H oder dessen Gemische nach Anspruch 1.

**16.** Zusammensetzung gemäß zumindest einem der Ansprüche 1 bis 15 enthaltend in der Summe weniger als 5 Gew.%, insbesondere weniger als 2 Gew.%, Lösungs- oder Verdünnungsmittel.

**17.** Verwendung von Salzen von Kohlenwasserstoffpolyalkoxysulfaten der Formel

$R^4$-[EO, PO, AO]-O-$SO_3^-$ $HNR^1R^2R^{3+}$

zur Behandlung von Erdöllagerstätten oder zur Gewinnung von Kohlenwasserstoffen aus Teersänden oder anderen mit Kohlenwasserstoff versehenen Oberflächen oder Materialien, wobei

• die Anzahl n der Alkoxygruppen EO, PO, AO zusammen 1 bis 16, beträgt
und die Alkoxygruppen ausgewählt sind aus:

- 1 bis 16 oder 4 bis 13, Propoxygruppen (PO)
und ggf. einer oder mehrerer der Gruppen
- 0 bis 15 Ethoxygruppen (EO), und
- 0 bis 5 C4 bis C12 Alkylenoxygruppen (AO),
und die Gruppen statistisch verteilt sind oder in Blöcken vorliegen oder beides; und

• ein, zwei oder drei der Reste $R^1$, $R^2$ und $R^3$ sind unabhängig voneinander ausgewählt aus der Gruppe:

- Alkyl mit 1 bis 14 Kohlenstoffatomen,
- Alkenyl mit 3 bis 18 Kohlenstoffatomen,
- Hydroxyalkyl mit 3 oder 4 Kohlenstoffatomen,
- zwei oder drei Reste sind Hydroxyalkly mit 2 Kohlenstoffatomen,
- und deren Gemischen,

wobei das Hydroxyalkyl ggf. alkoxyliert ist, und
wobei die verbleibenden Reste jeweils Wasserstoff sind; und
• $R^4$ ein oder mehrere unterschiedliche C10 bis C36 Kohlenwasserstoffe sind.

**18.** Verwendung gemäß Anspruch 17, worin $R^4$ für ein oder mehrere unterschiedliche C12 bis C36 Kohlenwasserstoffe, insbesondere C12 bis C24 Kohlenwasserstoffe steht.

**19.** Verwendung gemäß zumindest einem der Ansprüche 17 oder 18, wobei zwei oder drei der Reste $R^1$, $R^2$ und $R^3$ Hydroxyalkyl mit jeweils 2 und/oder 3 Kohlenstoffatomen sind und die verbleibenden Reste jeweils Wasserstoff, wobei das C2 oder C3 Hydroxyalkyl ggf. alkoxyliert ist.

**20.** Verwendung gemäß zumindest einem der Ansprüche 17 bis 19, wobei ein, zwei oder drei der Reste $R^1$, $R^2$ und $R^3$ jeweils Isopropanol sind, vorzugsweise ein oder drei der Reste $R^1$, $R^2$ und $R^3$, und die verbleibenden Reste jeweils

Wasserstoff sind, wobei das Isopropanol ggf. alkoxyliert ist.

**21.** Verwendung gemäß zumindest einem der Ansprüche 17 bis 20, wobei die Anzahl n der Alkoxygruppen EO, PO, AO zusammen 3 bis 16 vorzugsweise 4 bis 13 oder 3 bis 10 beträgt.

**22.** Verwendung gemäß zumindest einem der Ansprüche 17 bis 21, wobei die EO, PO, AO Alkoxygruppen unabhängig voneiander ausgewählt sind aus:

- 4 bis 13 Propoxygruppen (PO),
- 1 bis 8 oder 1 bis 3, Ethoxygruppen (EO), und
- 0 bis 5 oder 1 bis 3, C4 bis C12 Alkylenoxygruppen (AO), insbesondere Butoxygruppen,

und die Alkoxygruppen statistisch verteilt sind oder in Blöcken vorliegen oder beides.

**23.** Verwendung nach Anspruch 17, wobei die Salze der Kohlenwasserstoffpolyalkoxysulfate als Teil von Zusammensetzungen gemäß einem der Ansprüche 1 bis 16 durch Verdünnung mit Wasser erhältlich sind.

**24.** Verfahren zur Einbringung von wässrigen Zusammensetzungen enthaltend 0,05 bis 5 Gew.% Kohlenwasserstoffpolyalkoxysulfate der Formel

$$R^4\text{-[EO, PO, AO]-O-}SO_3^- \; HNR^1R^2R^{3+},$$

wobei

• die Anzahl der Alkoxygruppen EO, PO, AO zusammen 1 bis 70, bevorzugt 1 bis 30, besonders bevorzugt 3 bis 16, beträgt, und die Alkoxygruppen ausgewählt sind aus:

- 1 bis 60, besonders 1 bis 30, insbesondere 3 bis16 oder 4 bis 13, Propoxygruppen (PO),
und ggf. einer oder mehrerer der Gruppen
- 0 bis 70, insbesondere 0 bis 40, vorzugsweise 0 bis 15, besonders bevorzugt 0 bis 8 oder alternativ 1 bis 3, Ethoxygruppen (EO), und
- 0 bis 25, insbesondere 0 bis15, vorzugsweise 0 bis 10, besonders bevorzugt 0 bis 5 oder alternativ 1 bis 3, C4 bis C12 Alkylenoxygruppen (AO), insbesondere Butoxygruppen,
und die Gruppen statistisch verteilt sind oder in Blöcken vorliegen oder beides,

in unterirdische Erdöl-Lagerstätten zur Unterstützung der Förderung von Erdöl.
• ein, zwei oder drei der Reste $R^1$, $R^2$ und $R^3$ sind unabhängig voneinander Alkyl mit 1 bis 14, insbesondere mit 4 bis 8 Kohlenstoffatomen, Alkenyl mit 3 bis 18, insbesondere 4 bis 8, Kohlenstoffatomen und/oder Hydroxyalkyl mit 1 bis 4, insbesondere 2 oder 3, Kohlenstoffatomen, wobei das Hydroxyalkyl ggf. alkoxyliert ist, wobei die verbleibenden Reste unabhängig voneinander Wasserstoff sind, und
• • $R^4$ ein oder mehrere unterschiedliche C10 bis C36 Kohlenwasserstoffe sind, insbesondere C12 bis C24, besonders bevorzugt C10 bis C18,

**25.** Verfahren gemäß Anspruch 24, weiter **gekennzeichnet durch** eines oder mehrere der folgenden Merkmale:

a) worin $R^4$ für ein oder mehrere unterschiedliche C12 bis C36 Kohlenwasserstoffe, insbesondere C12 bis C24 Kohlenwasserstoffe steht;
b) wobei zwei oder drei der Reste $R^1$, $R^2$ und $R^3$ Hydroxyalkyl mit jeweils 2 und/oder 3 Kohlenstoffatomen sind und die verbleibenden Reste jeweils Wasserstoff, wobei das C2 oder C3 Hydroxyalkyl ggf. alkoxyliert ist;
c) wobei ein, zwei oder drei der Reste $R^1$, $R^2$ und $R^3$ jeweils Isopropanol sind, vorzugsweise ein oder drei der Reste $R^1$, $R^2$ und $R^3$, und die verbleibenden Reste jeweils Wasserstoff sind, wobei das Isopropanol ggf. alkoxyliert ist;
d) wobei die Anzahl n der Alkoxygruppen EO, PO, AO zusammen 3 bis 16 vorzugsweise 4 bis 13 oder 3 bis 10 beträgt;
e) wobei die EO, PO, AO Alkoxygruppen unabhängig voneinander ausgewählt sind aus:

- 4 bis 13 Propoxygruppen (PO),
- 1 bis 8 oder 1 bis 3, Ethoxygruppen (EO), und

- 0 bis 5 oder 1 bis 3, C4 bis C12 Alkylenoxygruppen (AO), insbesondere Butoxygruppen,

und die Alkoxygruppen statistisch verteilt sind oder in Blöcken vorliegen oder beides;
f) wobei die Zusammensetzungen 0,1 bis 3 Gew.% der Kohlenwasserstoffpolyalkoxysulfate enthält;
g) wobei die unterirdischen Erdöl-Lagerstätten Reservoirtemperaturen des zu behandelnden Reservoirs von 0°C bis 100°C, vorzugsweise von 10°C bis 80°C, insbesondere von 15°C bis 70°C, aufweisen;
h) wobei die Erdöl-Lagerstätten Reservoirtemperaturen des zu behandelnden Reservoirs von im Maximum 75 bis 110°C aufweisen;
i) wobei die Salze der Kohlenwasserstoffpolyalkoxysulfate als Teil von Zusammensetzungen gemäß einem der Ansprüche 1 bis 17 durch Verdünnung mit Wasser erhältlich sind.

## Claims

**1.** A highly concentrated composition of amine salts of hydrocarbon polyalkoxy sulfates that is flowable at 25 °C, comprising:

(A) greater than 80% by weight, in particular greater than 90% by weight and in particular greater than 95% by weight amine salts of hydrocarbon polyalkoxy sulfates of the formula

$$R^4\text{-O-[EO, PO, AO]}_n\text{- }SO_3^-\ HNR^1R^2R^{3+}$$

wherein
one, two or three of the substituents $R^1$, $R^2$ and $R^3$ are each independently selected from the group:

- alkyl having 1 to 14 carbon atoms,
- alkenyl having 3 to 18 carbon atoms,
- hydroxyalkyl having 3 or 4 carbon atoms,
- two or three substituents are hydroxyalkyl having 2 carbon atoms,
- and mixtures thereof,

wherein the hydroxyalkyl is optionally alkoxylated and
wherein the remaining substituents are each hydrogen; and

- $R^4$ is/are one or more different C10 to C36 hydrocarbon(s);

optionally in a mixture with less than 20% by weight, in particular less than 10% by weight of said above amine salts of hydrocarbon polyalkoxy sulfates in which said alkoxy groups are exclusively EO;
(B) at least 0.1 up to less than 5% by weight, in particular 0.2 to 3% by weight, of the non-sulfated polyalkoxylated hydrocarbon $R^4$-O-[EO, PO, AO]$_n$- H or mixtures;
(C) up to less than 5% by weight of said non-sulfated hydroxy hydrocarbons $R^4$-OH or of the mixture;
wherein (B) and (C) together represent 0.1 to 10 % by weight, in particular 0.5 to 5 % by weight, of the composition;

wherein the composition
comprises less than 2 % by weight of water and
the number n of said alkoxy groups EO, PO, AO combined is 1 to 16,

- and the alkoxy groups are selected from:
- 1 to 16, propoxy groups (PO)
- and optionally from one or more of the groups
- 0 to 15 ethoxy groups (EO), and
- 0 to 10 C4 to C12 alkyleneoxy groups (AO),

and the alkoxy groups are distributed randomly or are present in blocks, or both.
and the alkoxy groups are distributed randomly or are present in blocks, or both.

**2.** The composition according to claim 1, wherein $R^4$ is one or more different C12 to C36 hydrocarbon atoms, in particular C12 to C24 hydrocarbon atoms.

**3.** The composition according to at least one of the claims 1 or 2, wherein two or three of the substituents $R^1$, $R^2$ and $R^3$ are hydroxyalkyl each having 2 and/or 3 carbon atoms and the remaining substituents are each hydrogen, wherein the C2 or C3 hydroxyalkyl optionally is alkoxylated.

**4.** The composition according to at least one of the claims 1 to 3, wherein one, two or three of the substituents $R^1$, $R^2$ and $R^3$ each are isopropanol, preferably one or three of the substituents $R^1$, $R^2$ and $R^3$, and the remaining substituents are each hydrogen, wherein the isopropanol optionally is alkoxylated.

**5.** The composition according to at least one of the claims 1 to 4, wherein the number n of the alkoxy groups EO, PO, AO combined is 3 to 16, preferably 4 to 13 or 3 to 10.

**6.** The composition according to at least one of the claims 1 to 5, wherein the EO, PO, AO alkoxy groups are selected independently from each other from:

- 4 to 13 propoxy groups (PO),
- 1 to 8 or 1 to 3 ethoxy groups (EO), and
- 0 to 5 or 1 to 3 C4 to C12 alkyleneoxy groups (AO), in particular butoxy groups, and the alkoxy groups are distributed randomly or are present in blocks, or both.

**7.** The composition according to at least one of the claims 1 to 6, wherein the composition consists out of the components (A) to (C) and optionally water.

**8.** The composition according to at least one of the claims 1 to 7, comprising less than 0.5% by weight water, more preferably less than 0.05% by weight water.

**9.** The composition according to at least one of claims 1 to 8, further comprising

(D) up to 5% by weight, in particular 0.1 to 5% by weight, of the non-sulfated amine or of the amine mixture $NR^1R^2R^3$.

**10.** The composition according to at least one of the claims 1 to 9, further comprising

(E) 0.1 to 5 % by weight, in particular 0.1 to 2% by weight, of the sulfate of the amine or of the amine mixture $(HNR^1R^2R^3)_2SO_4$.

**11.** The composition according to at least one of the claims 1 to 10, further comprising

(F) 0.1 to 5% by weight, in particular 0.5 to 2% by weight

$$HNR^1R^2R^{3+}\ {}^-O_3SO\text{-[EO, PO, AO]-}SO_3^-\ HNR^1R^2R^{3+}.$$

**12.** The composition according to at least one of the claims 1 to 11, further comprising 0.05 to 10% by weight, in particular 0.1 to 5% by weight of the sulfated non-alkoxylated hydroxy hydrocarbon $R^4$-O-$SO_3^-$ $HNR^1R^2R^{3+}$ or mixtures thereof.

**13.** The composition according to at least one of the claims 1 to 12, wherein the composition is flowable at 15 °C and above.

**14.** The composition according to at least one of the claims 1 to 13, wherein the composition has a pour point of less than +5 °C, preferably less than -5 °C.

**15.** The composition according to at least one of the claims 1 to 14, comprising in total less than 10% by weight, preferably less than 3% by weight, of other nonionic surfactants except $R^4$-O-[EO, PO, AO]$_n$- H and mixtures thereof according to claim 1.

**16.** The composition according to at least one of the claims 1 to 15, further comprising in total less than 5% by weight, in particular less than 2% by weight, of solvent or diluent.

**17.** A use of salts of hydrocarbon polyalkoxy sulfates of the formula

$R^4$ [EO, PO, AO]-O-$SO_3^-$ $HNR^1R^2R^{3+}$

for the treatment of crude oil reservoirs or for the recovery of hydrocarbons from tar sands or other surfaces or materials provided with hydrocarbon, wherein

- the number n of said alkoxy groups EO, PO, AO combined is 1 to 16,
and said alkoxy groups are selected from:

  - 1 to 16 or 4 to 13 propoxy groups (PO),
  and optionally one or more of the groups
  - 0 to 15 ethoxy groups (EO), and
  - 0 to 5 C4 to C 12 alkyleneoxy groups (AO),

and the groups are distributed randomly or are present in blocks, or both; and
- one, two or three of the substituents $R^1$, $R^2$ and $R^3$ are each independently selected from the group:

  - alkyl having 1 to 14 carbon atoms,
  - alkenyl having 3 to 18 carbon atoms,
  - hydroxyalkyl having 3 or 4 carbon atoms,
  - two or three substituents are hydroxyalkyl having 2 carbon atoms,
  - and mixtures thereof,

wherein said hydroxyalkyl is optionally alkoxylated; and
wherein the remaining substituents are each hydrogen; and
- $R^4$ is one or more different C10 to C36 hydrocarbons.

**18.** The use according to claim 17, wherein $R^4$ is one or more different C12 to C36 hydrocarbons, in particular C12 to C24 hydrocarbons.

**19.** The use according to at least one of the claims 17 or 18, wherein two or three of the substituents $R^1$, $R^2$ and $R^3$ are hydroxyalkyl each having 2 and/or 3 carbon atoms and the remaining substituents are each hydrogen, wherein the C2 or C3 hydroxyalkyl optionally is alkoxylated.

**20.** The use according to at least one of the claims 17 to 19, wherein one two or three of the substituents $R^1$, $R^2$ and $R^3$ each are isopropanol, preferably one or three of the substituents $R^1$, $R^2$ and $R^3$, and the remaining substituents are each hydrogen, wherein the isopropanol optionally is alkoxylated.

**21.** The use according to at least one of the claims 17 to 20, wherein the number n of the alkoxy groups EO, PO, AO combined is 3 to 16, preferably 4 to 13 or 3 to 10.

**22.** The use according to at least one of the claims 17 to 21, wherein the EO, PO, AO alkoxy groups are selected independently from each other from:

- 4 to 13 propoxy groups (PO),
- 1 to 8 or 1 to 3 ethoxy groups (EO), and
- 0 to 5 or 1 to 3 C4 to C12 alkyleneoxy groups (AO), in particular butoxy groups,

and the alkoxy groups are distributed randomly or are present in blocks, or both.

**23.** The use according to claim 17, wherein said salts of hydrocarbon polyalkoxy sulfates are available as part of compositions according to one of claims 1 to 16 by dilution with water.

**24.** A method for the introduction of aqueous compositions comprising from 0.05 to 5% by weight of hydrocarbon polyalkoxy sulfates of the formula

$R^4$-[EO, PO, AO]-O-$SO_3^-$ $HNR^1R^2R^{3+}$,

wherein

- the number of said alkoxy groups EO, PO, AO combined is 1 to 70, preferably 1 to 30, most preferably 3 to 16, and said alkoxy groups are selected from:

  - 1 to 60, in particular 1 to 30, especially in particular 3 to 16 or 4 to 13 propoxy groups (PO), and optionally one or more of the groups
  - 0 to 70, in particular 0 to 40, preferably 0 to 15, most preferably 0 to 8 or alternatively 1 to 3 ethoxy groups (EO), and
  - 0 to 25, in particular 0 to 15, preferably 0 to 10, most preferably 0 to 5 or alternatively 1 to 3 C4 to C12 alkyleneoxy groups (AO), in particular butoxy groups, and the groups are distributed randomly or are present in blocks, or both,

- one, two or three of substituents $R^1$, $R^2$ and $R^3$ are each independently alkyl having 1 to 14, in particular 4 to 8 carbon atoms, alkenyl having 3 to 18, in particular 4 to 8 carbon atoms and/or hydroxyalkyl having 1 to 4, in particular 2 or 3 carbon atoms, wherein said hydroxyalkyl is optionally alkoxylated,
wherein the remaining substituents are independent from each other hydrogen, and
- $R^4$ is one or more different C10 to C36 hydrocarbons, in particular C12 to C24, most preferably C 10 to C 18,

into underground crude oil reservoirs to support the production of crude oil.

**25.** The method according to claim 24, further **characterized by** one or more of the following features:

a) wherein $R^4$ is one or more different C12 to C36 hydrocarbons, in particular C12 to C24 hydrocarbons;
b) wherein two or three of the substituents $R^1$, $R^2$ and $R^3$ are hydroxyalkyl each having 2 and/or 3 carbon atoms and the remaining substituents are hydrogen, wherein the C2 or C3 hydroxyalkyl optionally is alkoxylated;
c) wherein one, two or three of the substituents $R^1$, $R^2$ and $R^3$ each are isopropanol, preferably one or three of the substituents $R^1$, $R^2$ and $R^3$, and the remaining substituents are each hydrogen, wherein the isopropanol optionally is alkoxylated;
d) wherein the number n of the alkoxy groups EO, PO, AO combined is 3 to 16, preferably 4 to 13 or 3 to 10;
e) wherein the EO, PO, AO alkoxy groups are selected independently from each other from:

  - 4 to 13 propoxy groups (PO),
  - 1 to 8 or 1 to 3 ethoxy groups (EO), and
  - 0 to 5 or 1 to 3, C4 to C12 alkyleneoxy groups (AO), in particular butoxy groups, and the alkoxy groups are distributed randomly or are present in blocks, or both;

f) wherein the compositions comprise 0,1 % per weight to 3% per weight of the hydrocarbon polyalkoxy sulfates;
g) wherein the underground crude oil reservoirs have reservoir temperatures of the to be treated reservoir from 0 °C to 100 °C, preferably from 10 °C to 80 °C, in particular from 15 °C to 70 °C;
h) wherein the underground crude oil reservoirs have reservoir temperatures of the treated reservoir having a maximum of 75 °C to 110 °C;
i) wherein the salts of said hydrocarbon polyalkoxy sulfates are available as part of compositions according to one of claims 1 to 17 by dilution with water.

## Revendications

**1.** Composition très concentrée et écoulable à 25°C, de sels d'amines de polalkoxysulfates d'hydrocarbures, comprenant :

(A) plus de 80 % en poids, en particulier plus de 90 % en poids, notamment plus de 95 % en poids, de sels d'amines de polalkoxysulfates d'hydrocarbures de formule

$$R^4\text{-O-[EO, PO, AO]}_n\text{-}SO_3^{-}HNR^1R^2R^{3+}$$

dans laquelle

• un, deux ou trois des résidus $R^1$, $R^2$, et $R^3$ sont choisis, indépendamment les uns des autres, dans le groupe :

- alkyle ayant de 1 à 14 atomes de carbone,
- alkényle ayant de 3 à 18 atomes de carbone,
- hydroxyalkyle ayant de 3 à 4 atomes de carbone,
- deux ou trois des résidus sont hydroxyalkyle ayant de 2 atomes de carbone
- et leurs mélanges,

où l'hydroxyalkyle est le cas échéant alkoxylé et
où les résidus restants sont respectivement hydrogène ; et
• $R^4$ est/sont un ou plusieurs hydrocarbures en C10 à C36 différents ;

le cas échéant en mélange avec moins de 20 % en poids, en particulier moins de 10 % en poids, des sels d'amines ci-dessus de polalkoxysulfates d'hydrocarbures, dans lesquels les groupes alkoxy sont exclusivement EO ;
(B) au moins 0,1 à moins de 5 % en poids, en particulier 0,2 à 3 % en poids de l'hydrocarbure polyalkoxylé non sulfaté $R^4$-O-[EO, PO, AO]$_n$-H ou son mélange ;
(C) jusqu'à moins de 5 % en poids de l'hydrocarbure hydroxy non sulfaté $R^4$-OH ou son mélange ;
dans laquelle (B) et (C) constituent conjointement 0,1 à 10 % en poids, en particulier 0,5 à 5 % en poids, de la composition ;

ladite composition présentant moins de 2 % en poids d'eau et
dans laquelle le nombre n des groupes alkoxy EO, PO, AO conjointement est de 1 à 16 et les groupes alkoxy sont choisis de:

- 1 à 16 groupes propoxy (PO)
et le cas échéant un ou plusieurs groupes
- 0 à 15 groupes éthoxy (EO), et
- 0 à 10 groupes alkylènoxy en C4 à C12 (AO),

et les groupes alkoxy sont distribués statistiquement ou se présentent sous forme de blocs ou les deux.

**2.** Composition selon la revendication 1, dans laquelle $R^4$ représente un ou plusieurs hydrocarbures en C12 à C36 différents, en particulier des hydrocarbures en C12 à C24.

**3.** Composition selon au moins l'une des revendications 1 ou 2, dans laquelle deux ou trois des résidus $R^1$, $R^2$, et $R^3$ sont hydroxyalkyle ayant respectivement 2 et/ou 3 atomes de carbone et les résidus restants sont respectivement hydrogène, où l'hydroxyalkyle en C2 ou C3 est le cas échéant alkoxylé.

**4.** Composition selon au moins l'une des revendications 1 à 3, dans laquelle un, deux ou trois des résidus $R^1$, $R^2$, et $R^3$ sont respectivement isopropanol, de préférence un ou trois des résidus $R^1$, $R^2$, et $R^3$, et les résidus restants sont respectivement hydrogène, où l'isopropanol est le cas échéant alkoxylé.

**5.** Composition selon au moins l'une des revendications 1 à 4, dans laquelle le nombre n des groupes alkoxy EO, PO, AO conjointement est de 3 à 16, de préférence de 4 à 13 ou 3 à 10.

**6.** Composition selon au moins l'une des revendications 1 à 5, dans laquelle les groupes alkoxy EO, PO, AO sont choisis, indépendamment les uns des autres parmi :

- 4 à 13 groupes propoxy (PO),
- 1 à 8 ou 1 à 3 groupes éthoxy (EO), et
- 0 à 5 ou 1 à 3 groupes alkylènoxy en C4 à C12 (AO), en particulier groupes butoxy, et

les groupes alkoxy sont distribués statistiquement ou se présentent sous forme de blocs ou les deux.

**7.** Composition selon au moins l'une des revendications 1 à 6, ladite composition se composant des composants (A) à (C) et le cas échéant d'eau.

**8.** Composition selon au moins l'une des revendications 1 à 7, dans laquelle la composition comprend moins de 0,5 % en poids d'eau, particulièrement préférentiellement moins de 0,05 % en poids d'eau.

**9.** Composition selon au moins l'une des revendications 1 à 8, contenant en outre

(D) jusqu'à 5 % en poids, en particulier 0,1 à 5 % en poids, de l'amine non sulfatée et/ou du mélange d'amines $NR^1R^2R^3$.

**10.** Composition selon au moins l'une des revendications 1 à 9, contenant en outre

(E) de 0,1 à 5 % en poids, en particulier 0,1 à 2 % en poids, du sulfate d'amine et/ou du mélange d'amines $(HNR^1R^2R^3)_2SO_4$.

**11.** Composition selon au moins l'une des revendications 1 à 10, contenant en outre

(E) de 0,1 à 5 % en poids, en particulier 0,5 à 2 % en poids, de $HNR^1R^2R^{3+}$-$O_3SO$-[EO, PO, AO]$_n$-$SO_3^-$ $HNR^1R^2R^{3+}$.

**12.** Composition selon au moins l'une des revendications 1 à 11, contenant en outre 0,05 à 10 % en poids, en particulier 0,1 à 5 % en poids, de l'hydrocarbure hydroxy non alkoxylé sulfaté $R^4$-O-$SO_3^-HNR^1R^2R^{3+}$ ou de mélanges.

**13.** Composition selon au moins l'une des revendications 1 à 12, ladite composition étant écoulable à 15°C et plus.

**14.** Composition selon au moins l'une des revendications 1 à 13, ladite composition présentant un point d'écoulement inférieur à +5°C, de préférence inférieur à -5°C.

**15.** Composition selon au moins l'une des revendications 1 à 14, contenant au total moins de 10 % en poids, de préférence moins de 3 % en poids, d'autres tensioactifs non ioniques à l'exception des $R^4$-O-[EO, PO, AO]$_n$-H ou son mélange selon la revendication 1.

**16.** Composition selon au moins l'une des revendications 1 à 15, contenant au total moins de 5 % en poids, notamment moins de 2 % en poids de solvant ou diluant.

**17.** Utilisation de sels de polalkoxysulfates d'hydrocarbures de formule

$$R^4\text{-[EO, PO, AO]-O-}SO_3^-\ HNR_1R^2R^{3+}$$

pour le traitement de gisements pétroliers ou pour l'extraction d'hydrocarbures à partir de sables bitumeux ou autres surfaces ou matières pourvues d'hydrocarbure, dans laquelle

• le nombre n des groupes alkoxy EO, PO, AO conjointement est de 1 à 16 et les groupes alkoxy sont choisis:

- 1 à 16 ou 4 à 13 groupes propoxy (PO)
et le cas échéant un ou plusieurs groupes
- 0 à 15 groupes éthoxy (EO), et
- 0 à 5 groupes alkylènoxy en C4 à C12 (AO),

et les groupes sont distribués statistiquement ou se présentent sous forme de blocs ou les deux ; et
• un, deux ou trois des résidus $R^1$, $R^2$, et $R^3$ sont choisis, indépendamment les uns des autres, dans le groupe :

- alkyle ayant de 1 à 14 atomes de carbone,
- alkényle ayant de 3 à 18 atomes de carbone,
- hydroxyalkyle ayant de 3 ou 4 atomes de carbone,
- deux ou trois des résidus sont hydroxyalkyle ayant 2 atomes de carbone,
- et leurs mélanges,

dans laquelle l'hydroxyalkyle est le cas échéant alkoxylé, et
dans laquelle les résidus restants sont respectivement hydrogène ; et

• $R^4$ sont un ou plusieurs hydrocarbures en C10 à C36 différents..

**18.** Utilisation selon la revendication 17, dans laquelle $R^4$ représente un ou plusieurs hydrocarbures en C 12 à C36 différents, en particulier des hydrocarbures en C12 à C24.

**19.** Utilisation selon au moins l'une des revendications 17 ou 18, dans laquelle deux ou trois des résidus $R^1$, $R^2$, et $R^3$ sont hydroxyalkyle ayant respectivement 2 et/ou 3 atomes de carbone et les résidus restants sont respectivement hydrogène, où l'hydroxyalkyle en C2 ou C3 est le cas échéant alkoxylé.

**20.** Utilisation selon au moins l'une des revendications 17 à 19, dans laquelle un, deux ou trois des résidus $R^1$, $R^2$, et $R^3$ sont respectivement isopropanol, de préférence un ou trois des résidus $R^1$, $R^2$, et $R^3$, et les résidus restants sont respectivement hydrogène, où l'isopropanol est le cas échéant alkoxylé.

**21.** Utilisation selon au moins l'une des revendications 17 à 20, dans laquelle le nombre n des groupes alkoxy EO, PO, AO conjointement est de 3 à 16, de préférence de 4 à 13 ou 3 à 10.

**22.** Utilisation selon au moins l'une des revendications 17 à 21, dans laquelle les groupes alkoxy EO, PO, AO sont choisis indépendamment l'un de l'autre parmi :

- 4 à 13 groupes propoxy (PO),
- 1 à 8 ou 1 à 3 groupes éthoxy (EO), et
- 0 à 5 ou 1 à 3 groupes alkylènoxy en C4 à C12 (AO), en particulier groupes butoxy,

et les groupes alkoxy sont distribués statistiquement ou se présentent sous forme de blocs ou les deux.

**23.** Utilisation selon la revendication 17, dans laquelle les sels de polyalkoxysulfates d'hydrocarbures peuvent être obtenus sous forme de partie des compositions selon l'une des revendications 1 à 16 par dilution avec de l'eau.

**24.** Procédé d'introduction de compositions aqueuses contenant 0,05 à 5 % en poids de polalkoxysulfates d'hydrocarbures de formule

$$R^4\text{-[EO, PO, AO]-O-}SO_3^- \ HNR^1R^2R^{3+},$$

dans laquelle

• le nombre n des groupes alkoxy EO, PO, AO conjointement est de 1 à 70, de préférence de 1 à 30, particulièrement préférentiellement 3 à 16,:

et les groupes alkoxy sont choisis:

- 1 à 60, en particulier 1 à 30, en particulier 3 à 16 ou 4 à 13 groupes propoxy (PO), et le cas échéant un ou plusieurs groupes
- 0 à 70, en particulier 0 à 40, en particulier 0 à 15, particulièrement préférentiellement 0 à 8 ou alternative 1 à 3, groupes éthoxy (EO), et
- 0 à 25, en particulier 0 à 15, de préférence 0 à 10, particulièrement préférentiellement 0 à 5 ou alternative 1 à 3, groupes alkylènoxy en C4 à C12 (AO), en particulier groupes butoxy,

et les groupes sont distribués statistiquement ou se présentent sous forme de blocs ou les deux ; et

• un, deux ou trois des résidus $R^1$, $R^2$, et $R^3$ sont, indépendamment les uns des autres, alkyle ayant de 1 à 14, en particulier 4 à 8 atomes de carbone, alkényle ayant de 3 à 18, en particulier 4 à 8 atomes de carbone et/ou hydroxyalkyle ayant de 1 à 4, en particulier 2 ou 3 atomes de carbone, où l'hydroxyalkyle est le cas échéant alkoxylé, et où les résidus restants sont indépendamment les uns des autres hydrogène, et
• $R^4$ sont un ou plusieurs hydrocarbures en C10 à C36 différents, en particulier des hydrocarbures en C12 à C24, particulièrement préférentiellement C10 à C18,

dans des gisements pétroliers souterrains pour fournir une aide dans l'extraction du pétrole.

**25.** Procédé selon la revendication 24, **caractérisé par** une ou plusieurs des caractéristiques suivantes :

a) $R^4$ représente un ou plusieurs hydrocarbures en C12 à C36 différents, de préférence des hydrocarbures en C12 à C24;
b) deux ou trois des résidus $R^1$, $R^2$, et $R^3$ sont hydroxyalkyle ayant respectivement 2 et/ou 3 atomes de carbone et les résidus restants sont respectivement hydrogène, où l'hydroxyalkyle en C2 ou C3 est le cas échéant alkoxylé ;
c) un, deux ou trois des résidus $R^1$, $R^2$, et $R^3$ sont respectivement isopropanol, de préférence un ou trois des résidus $R^1$, $R^2$, et $R^3$, et les résidus restants sont respectivement hydrogène, où l'isopropanol est le cas échéant alkoxylé ;
d) le nombre n des groupes alkoxy EO, PO, AO conjointement est de 3 à 16, de préférence de 4 à 13 ou 3 à 10 ;
e) les groupes alkoxy EO, PO, AO sont choisis indépendamment l'un de l'autre parmi :

- 4 à 13 groupes propoxy (PO),
- 1 à 8 ou 1 à 3 groupes éthoxy (EO), et
- 0 à 5 ou 1 à 3 groupes alkylènoxy en C4 à C12 (AO), en particulier groupes butoxy,

et les groupes alkoxy sont distribués statistiquement ou se présentent sous forme de blocs ou les deux ;
f) les compositions contiennent 0,1 à 3 % en poids de polyalkoxysulfates d'hydrocarbures ;
g) les gisements pétroliers souterrains présentent des températures de réservoir du réservoir à traiter de 0°C à 100°C, de préférence de 10°C à 80°C, en particulier de 15°C à 70°C ;
h) les gisements pétroliers souterrains présentent des températures de réservoir du réservoir à traiter de 75°C à 110°C au maximum ;
i) les sels de polyalkoxysulfates d'hydrocarbures peuvent être obtenus sous forme de partie des compositions selon l'une des revendications 1 à 17 par dilution avec de l'eau.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- GB 2168095 A
- WO 2009124922 A
- WO 2011110502 A1
- EP 0167337 A2
- EP 0656416 A1
- US 4477372 A
- US 4703797 A
- US 4017405 A


### In der Beschreibung aufgeführte Nicht-Patentliteratur


- **C. MELROSE ; CF. BRANDNER.** *J. Canadian Petr. Techn.,* Oktober 1974, vol. 58